# EUROPÄISCHE PATENTSCHRIFT

(11) **EP 1 088 530 B1**
(45) Veröffentlichungstag und Bekanntmachung des Hinweises auf die Patenterteilung: **28.06.2006**
(21) Anmeldenummer: 99118861.6
(22) Anmeldetag: 24.09.1999
(51) Int. Cl.: A61F 2/30

(54) **Alloplastischer Ersatz für einen langen Knochen**
Alloplastic prosthesis for long bone
Prothése alloplastique pour un os long

(43) Veröffentlichungstag der Anmeldung: 04.04.2001
(73) Patentinhaber: WALDEMAR LINK GmbH & Co. KG, 22339 Hamburg (DE)
(72) Erfinder: Keller, Arnold, 23863 Kayhude (DE)
(74) Vertreter: Glawe, Delfs, Moll

(56) Entgegenhaltungen:
- EP-A- 0 212 192
- EP-A- 0 621 019
- FR-A- 2 666 221
- US-A- 4 502 160
- US-A- 5 358 524

## Beschreibung

Die Erfindung bezieht sich auf einen alloplastischen Knochenersatz gemäß Oberbegriff des Anspruchs 1.

Wird ein langer Knochen eines noch nicht ausgewachsenen Jugendlichen ganz oder teilweise durch ein Implantat ersetzt, so ergeben sich Schwierigkeiten daraus, daß das Implantat dem Wachstum anderer Körperteile nicht zu folgen vermag. Wird beispielsweise das Schienbein oder ein Teil desselben wegen Tumorbefalls durch das Implantat ersetzt, so folgt dieses Implantat nicht dem Wachstum des Wadenbeins.

Es ist ein längenverstellbares Implantat bekannt (FR-A-2666221), bei dem eine Gewindespindel teilweise in einer Hülse steckt und die Einstecklänge durch eine auf der Spindel verschraubbare Mutter bestimmt wird. Durch Verdrehung der Mutter, deren axiale Position relativ zur Hülse festgelegt ist, läßt sich die Einstecklänge der Spindel verändern und den individuellen Erfordernissen anpassen. Hülse und Spindel sind mit einer Verdrehsicherung versehen, die eine Längsnut in der Spindel und in diese eingreifende Stifte an der Hülse umfaßt. Diese sind als Schrauben ausgebildet, die gegen den Nutgrund geschraubt werden, um eine axiale Feststelleinrichtung zu bilden, d. h. eine Einrichtung, die im Feststellzustand eine axiale Relativbewegung zwischen der Spindel und der Hülse verhindert. Es ist Aufgabe der vorliegenden Erfindung, die axiale Fixierung zu verbessern.

Dies wird erfindungsgemäß durch das kennzeichnende Merkmal des Anspruchs 1 und vorzugsweise die Merkmale der Unteransprüche gelöst. Demnach weist der Nutgrund eine Reihe von Vertiefungen auf und greift die Schraube in jeweils eine solche Vertiefung ein.

Die Schraube erfüllt zwei Funktionen: in einer ersten Einstellung greift sie lediglich lose in die Nut ein und erlaubt die axiale Verstellung, während sie in einer anderen Einstellung in eine Vertiefung des Nutgrundspreßt und dadurch die axiale Fixierung mit größerer Sicherheit als bei der bekannten Lösung bewirkt.

Die Mutter ist zweckmäßigerweise mit einer Verdrehsicherung versehen, die ebenfalls von einer Schraube gebildet sein kann, die in die Längsnut der Spindel eingreift.

Die erfindungsgemäße Anordnung ist Teil eines Implantats, das an beiden Enden an Skeletteile angeschlossen ist. Beispielsweise kann an einem Ende oder gewünschtenfalls an beiden Enden der Anordnung ein Schaft zur Verankerung in der Markhöhle eines Knochens vorgesehen sein. Dieser Schaft bedarf keiner näheren Erläuterung, da Schäfte zur Verankerung in Knochenhohlräumen bekannt sind. Ferner kann an einem Ende oder gewünschtenfalls an beiden Enden eine Gelenkkomponente vorgesehen sein, beispielsweise eine Kniegelenkkomponente an einem Ende und eine Knöchelgelenkkomponente am anderen. In vielen Fällen wird man es vorziehen, diese Gelenkkomponenten nicht unmittelbar und unlösbar mit der Anordnung zu verbinden, sondern statt dessen Kupplungsorgane an den Enden der Anordnung einerseits und an den Gelenkkomponenten andererseits vorzusehen, die unterschiedliche Kombination gestatten. Bei den Kupplungen kann es sich beispielsweise um Konuskupplungen handeln, die im Stand der Technik bekannt sind.

Die Erfindung wird im folgenden näher unter Bezugnahme auf die Zeichnung erläutert, die ein vorteilhaftes Ausführungsbeispiel veranschaulicht. Darin zeigen:

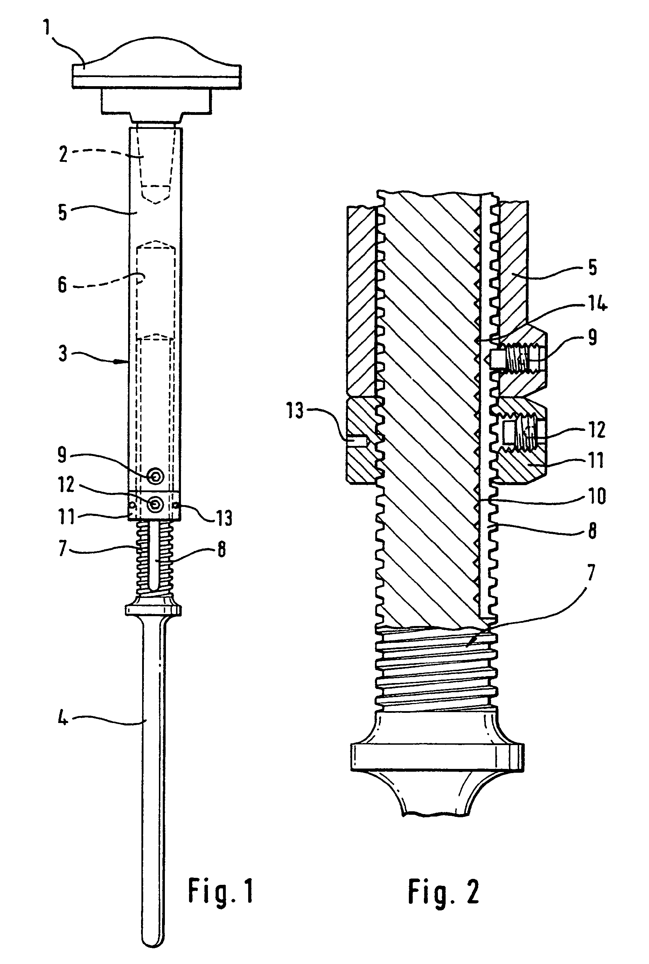

- Fig. 1: die Gesamtansicht eines Implantats und
- Fig. 2: eine geschnittene Teilansicht.

Mit der tibialen Komponente 1 einer Knieprothese ist über eine Konuskupplung 2 ein längenverstellbarer Teil 3 verbunden, dessen anderes Ende von einem Schaft 4 gebildet ist, der in der Markhöhle des Schienbeins zu verankern ist. Das Implantat ist dazu bestimmt, den oberen Teil des Schienbeins und die tibiale Komponente des Kniegelenks zu ersetzen.

Der längenverstellbare Teil 3 des Implantats besteht aus einer Hülse 5, die eine zylindrische Bohrung 6 enthält. Diese nimmt den oberen Abschnitt einer Gewindespindel 7 ohne wesentliches radiales Spiel und in Längsrichtung frei beweglich auf. Die Gewindespindel 7 enthält eine Längsnut 8. Am unteren Ende, d.h. der Kupplung 2 abgewandten Ende der Hülse 5 enthält diese eine radiale Gewindebohrung, die eine Feststellschraube 9 aufnimmt, die in die Nut 8 eingreift. Solange sie nicht gegen den Nutgrund 10 preßt, ist die Spindel 7 frei in der Hülse 5 in Längsrichtung verschiebbar, aber undrehbar.

Auf der Gewindespindel 7 sitzt eine Mutter 11 mit einer Feststellschraube 12, deren Anordnung der Feststellschraube 9 gleicht. Wenn sie in die Nut 8 eingreift, ist die Mutter gegenüber der Gewindespindel nicht drehbar. Will man die Mutter 11 verdrehen, zieht man die Schraube 12 aus der Nut 8 zurück.

Die Mutter 11 enthält verteilt über ihren gesamten Umfang einige Bohrungen 13, in die ein Stift eingesteckt werden kann, um die Mutter zu verdrehen. Sie wird bei der Längeneinstellung des Implantats stets so eingestellt, daß ihre Feststellschraube 12 in die Nut 8 eingreifen kann. Das bedeutet, daß die Mutter 11 auf der Gewindespindel 7 axiale Stellungen einnehmen kann, die sich jeweils um eine Ganghöhe unterscheiden. Daraus ergeben sich für die Hülse 5, deren unteres Ende sich an der Mutter 11 abstützt, ebenfalls diskrete Einstellungen gegenüber der Gewindespindel 7, die sich jeweils um eine Ganghöhe unterscheiden. Jeweils dort, wo sich in diesen Einstellungen die Spitze der Feststellschraube 9 befindet, enthält der Nutgrund 10 Vertiefungen 14. Die Feststellschraube 9 sichert die jeweils gewählten Einstellungen der Hülse daher nicht nur durch Reibkraft am Nutgrund 10, sondern auch durch formschlüssigen Eingriff in die Vertiefungen 14.

Bei der Längeneinstellung verfährt der Operateur folgendermaßen, wobei er dann, wenn es sich um einen Verlängerungsschritt handelt, sich zuvor durch einen Schnitt in das abdekkende Gewebe Zugang zu der Mutter 11 und der Feststellschraube 9 verschafft hat.

Die Feststellschraube 9 wird so gelöst, daß sie vom Nutgrund 10 und den darin enthaltenen Vertiefungen 14 frei ist, aber sich noch in der Nut 8 befindet. Die Feststellschraube 12 in der Mutter 11 wird gelöst und aus der Nut 8 zurückgezogen, so daß die Mutter 11 frei drehbar ist. Sie wird nun durch Eingriff des Stifts in die Bohrungen 13 schrittweise so verdreht, bis die gewünschte Einstellung erreicht ist, in welcher die Feststellschraube 12 wieder über der Nut 8 positioniert ist. In dieser Stellung werden die Feststellschrauben 9 und 12 wieder festgedreht, wodurch die Einstellung gesichert ist.

## Patentansprüche

1. Alloplastischer Ersatz für einen langen Knochen, der einen längenverstellbaren Teil (3) mit einer Gewindespindel (7), einer auf dieser verschraubbaren Mutter (11) und einer die Spindel (7) bis zur Mutter (11) aufnehmenden Hülse (5)umfaßt, wobei die Spindel (7) und die Hülse (5) eine Verdrehsicherung (8, 9), die eine Längsnut (8) in der Spindel (7) und einen in diese eingreifenden Eingreifteil an der Hülse (5) umfaßt, und eine axiale Feststelleinrichtung (9, 10, 14) aufweisen, die vom Nutgrund (10) der Nut (8) und dem als Schraube (9) ausgebildeten Eingreifteil gebildet wird, **dadurch gekennzeichnet, daß** der Nutgrund (10) der Nut (8) Vertiefungen (14) zum formschlüssigen Eingriff der Schraube (9) aufweist.

2. Knochenersatz nach Anspruch 1, **dadurch gekennzeichnet, daß** die Vertiefungen (14) einen gegenseitigen Abstand von einer Gewindeganghöhe aufweisen.

3. Knochenersatz nach Anspruch 1 oder 2, **dadurch gekennzeichnet, daß** auch die Mutter (11) mit einer Verdrehsicherung (8, 12) versehen ist.

4. Knochenersatz nach einem der Ansprüche 1 bis 3, **dadurch gekennzeichnet, daß** er an wenigstens einem Ende einen Schaft (4) zur Verankerung in einem Markkanal aufweist.

5. Knochenersatz nach einem der Ansprüche 1 bis 4, **dadurch gekennzeichnet, daß** er an wenigstens einem Ende mit einer Gelenkprothese (1) verbunden ist oder ein Kupplungselement (2) aufweist.

## Claims

1. Alloplastic replacement of a long bone comprising a length-adjustable part (3) with a threaded spindle (7), a nut (11) to be screwed on same, and a casing (5) which encompasses the spindle (7) all the way down to the nut (11), whereby spindle (7) and casing (5) display a locking device (8, 9) which comprises a longitudinal groove (8) in the spindle (7) and an insertion piece on the casing (5) engaging in it, also displaying an axial fastening device (9, 10, 14) which is constituted by the groove base (10) of the groove (8) and the insertion piece in the form of a screw (9), **characterized in that** the groove base (10) of the groove (8) displays notches (14) to achieve a form-fit insertion of the screw (9).

2. Bone replacement in accordance with Claim 1, **characterized in that** the notches (14) display a mutual distance of one thread lead.

3. Bone replacement in accordance with Claim 1 or 2, **characterized in that** the nut (11) is also equipped with a locking piston (8, 12).

4. Bone replacement in accordance with one of Claims from 1 to 3, **characterized in that** it displays a shaft (4) at least on one end for anchorage into the medullary canal.

5. Bone replacement in accordance with one of Claims from 1 to 4, **characterized in that** it is connected with a joint prosthesis (1) at least on one end, or displays a coupling element (2).

## Revendications

1. Remplacement alloplastique d'un os long qui comprend un élément ajustable en longueur (3) avec une tige filetée (7), un écrou à visser sur cette tige (11) ainsi qu'un manchon (5) pour le logement de la tige (7) jusqu'à l'écrou (11), en ce faisant que la tige (7) et le manchon (5) présentent un limiteur de torsion (8, 9), qui comprend une rainure longitudinale (8) dans la tige (7) et un élément s'y emboîtant dans le manchon (5) ainsi qu'un dispositif de fixation axiale (9, 10, 14), qui est formé par le fond de rainure (10) de la rainure (8) et l'élément d'emboîtement sous forme de vis (9), **caractérisé par le fait que** le fond de rainure (10) de la rainure (8) présente des évidements (14) pour l'engagement positif de la vis (9).

2. Os synthétique selon revendication 1 mais **caractérisé** du fait que les évidements (14) présentent un écart réciproque d'un pas de filet.

3. Os synthétique selon revendication 1 ou 2 **caractérisé** du fait que l'écrou (11) est également doté d'un limiteur de torsion (8, 12).

4. Os synthétique selon une des revendications de 1 à 3 **caractérisé** du fait qu'un moins une extrémité présente une tige (4) d'ancrage dans le canal de la moelle.

5. Os synthétique selon une des revendications de 1 à 4 **caractérisé** du fait qu'un moins une extrémité est reliée à une articulation prothétique (1) ou présente un élément d'accouplement (2).
